# EUROPEAN PATENT APPLICATION

(11) **EP 1 852 701 A1**
(43) Date of publication of application: **07.11.2007**
(21) Application number: 05719367.4
(22) Date of filing: 21.02.2005
(51) Int. Cl.: G01N 33/53, C12P 21/02

(54) **METHOD OF DETECTING REACTION OF PROTEIN WITH SAMPLE**

(71) Applicant: Olympus Corporation, Tokyo 151-0072 (JP)
(72) Inventor: OKAMOTO, Naoaki, c/o Intellectual Property Department, Hachioji-shi, Tokyo (JP)
(74) Representative: von Hellfeld, Axel
(86) International application number: PCT/JP2005/002748
(87) International publication number: WO 2006/087821

(57) **Abstract**

There is provided a method for detecting a reaction of a fluorescently labeled protein and a sample simply, in a short time and at a higher precision.

By using an expression system such as an extracellular gene expression system and an extracellular transcription or translation system, a reaction of expressing a protein from a constructed vector is performed. At this time, a fluorescently labeled amino acid is introduced into the expression system in the form of a tRNA having the fluorescently labeled amino acid. With expression of a protein, the fluorescently labeled amino acid is incorporated into the protein, and a fluorescently labeled protein (hereafter, referred to as fluorescently labeled protein) is produced. The fluorescently labeled protein and a sample S are mixed to prepare a mixed solution, and FCS measurement is performed. Based on a measured value of the FCS measurement, the presence or absence of a reaction is detected.

## Description

### Technical Field

The present invention relates to a method for detecting a reaction of a protein and a sample.

### Background Art

Jpn. Pat. Appln. KOKAI Publication No. 2000-139468 describes synthesis of a protein in a cell-free translation system or a viable cell system using, as a template, a product transcribed from DNA consisting of a coding region from which a termination codon has been deleted, under the control of a promoter region, in the presence of a labeling reagent composed of a labeling part consisting of a labeling substance, and an acceptor part consisting of a compound having the ability to bind to a C-terminal of the protein.

Japanese Patent No. 2953783 describes an effective method for identifying a drug which is active at a gene transcription level.

Jpn. Pat. Appln. KOKAI Publication No. 2001-321199 describes a method for quantitating a DNA binding protein in a biological sample.

Jpn. Pat. Appln. KOKAI Publication No. 2003-88369 describes a method for detecting the DNA endonuclease activity utilizing fluorescence correlation spectroscopy (FCS), and a relationship between a magnitude of a molecular weight and a magnitude of a translational diffusion time.

Jpn. Pat. Appln. KOKAI Publication No. 2002-543414 describes a method for characterizing a fluorescent molecule or other particle in a sample, and describes that a translational diffusion time is obtained from fluorescence intensity multiple distribution analysis (FIMDA) and fluorescence autoconvoluted intensity distribution analysis (FACID).

### Disclosure of Invention

Among the aforementioned prior art, each detection method described in Japanese Patent No. 2953783, Jpn. Pat. Appln. KOKAI Publication Nos. 2001-321199 and 2003-88369 uses a radioisotope for detection of a specified sequence or a molecule having the specified sequence, and performs electrophoresis, and selection by immobilization of a molecule on a solid substrate. Therefore, there is a problem that a procedure is troublesome, and it takes time to obtain the detection result.

In addition, by using a method for making a protein to be incorporated or fused GFP or euro for labeling the protein, the protein is incorporated a large substance other than the protein, and therefore the original function of the protein could not be maintained. Therefore, there is a problem that, even when a reaction experiment is performed using such a protein, it is difficult to reproduce a reaction which is actually conducted in a living body, at a higher precision.

Japanese Patent No. 2953783, and Jpn. Pat. Appln. KOKAI Publication Nos. 2001-321199 and 2003-88369 do not describe use of fluorescence correlation spectroscopy (FCS), fluorescence intensity multiple distribution analysis (FIMDA) and fluorescence autoconvoluted intensity distribution analysis (FACID) for analyzing a protein.

Jpn. Pat. Appln. KOKAI Publication Nos. 2003-88369 and 2002-543414 do not describe a method for detecting a reaction of a fluorescently labeled protein and a sample.

An object of the present invention is to provide a method for detecting a reaction of a fluorescently labeled protein and a sample, simply, in a short time and at a higher precision.

In order to achieve the above object, the present invention is characterized by including: synthesizing a fluorescently labeled protein using an expression system which extracellularly expresses a protein, a vector having a gene encoding the protein incorporated therein, and a fluorescently labeled amino acid; mixing a solution containing the fluorescently labeled protein with a sample; and obtaining a size, brightness or count of a substance having a fluorescent label in the mixed solution by a fluorescence analysis method.

According to this feature, a protein can be fluorescently labeled without incorporating a large substance other than the protein or chemically modifying the protein, and therefore a reaction experiment can be performed while the original function of a protein is maintained, and the size, brightness or count of a substance having a fluorescent label can be obtained by mixing of each solution and using a fluorescence analysis method. Therefore, the detection result such as the presence or absence of a reaction, and change in the size, brightness, and number of protein can be obtained simply, in a short time and at a higher precision, without a troublesome procedure such as utilization of a radioisotope, electrophoresis, work of immobilizing a molecule on a solid substrate, and washing work.

Particularly, a reaction experiment can be performed in a homogeneous system in which a protein and a reactive substance are reacted with each other in a solution while being mixed, therefore transfer of a reagent can be automated, and reactors having a variety of forms can be used. Further, since a lot of samples can be handled on microplate at once, analysis can be performed countably, continuously, simply, in a short time and at a higher precision. In addition, since a solid support is not used, even a protein which is difficult to be solid-phased can be reacted and analyzed.

In addition, change in the size, brightness, and number of a reaction product in a floating system can be detected at an excellent sensitivity of a nM order by mixing of each solution and utilizing fluorescence correlation spectroscopy (FCS), fluorescence cross-correlation spectroscopy (FCCS), fluorescence intensity distribution analysis (FIDA), fluorescence intensity multiple distribution analysis (FIMDA) or FIDA-polarization.

The present invention is also characterized by including: synthesizing a fluorescently labeled protein using an expression system which extracellularly expresses a protein, a vector having a gene encoding the protein incorporated therein, and a fluorescently labeled amino acid; mixing a solution containing the fluorescently labeled protein, a sample and a substance which reacts with the protein; and obtaining a size, brightness or count of a substance having a fluorescent label in the mixed solution by a fluorescence analysis method.

According to this feature, a protein can be fluorescently labeled without introducing a large substance into the protein or chemically modifying the protein, a reaction experiment can be performed while the native function of the protein is maintained, and the size, brightness or count of a substance having a fluorescent label can be obtained by mixing of each solution and using a fluorescence analysis. Therefore, the detection result such as the presence or absence of a reaction, change in the size, brightness, or number of a protein, and promotion or inhibition of, or absence of influence on, a reaction by a substance reacting with a protein can be obtained simply, in a short time and at a higher precision, without a troublesome procedure such as utilization of a radioisotope, electrophoresis, immobilization of a molecule on a solid substrate, and washing work.

The present invention is also characterized by including: synthesizing a fluorescently labeled protein using an expression system which extracellularly expresses a protein, a vector having a gene encoding the protein incorporated therein, and a fluorescently labeled amino acid;
mixing a solution containing the fluorescently labeled protein with a sample to prepare a first mixed solution;
mixing the solution containing the fluorescently labeled protein, the sample and a substance which reacts with the protein to prepare a second mixed solution;
obtaining a size, brightness or count of a substance having a fluorescent label in the first mixed solution by a fluorescence analysis method;
obtaining a size, brightness or count of a substance having a fluorescent label in the second mixed solution by a fluorescence analysis method; and
detecting promotion or inhibition of, or absence of influence on, a reaction by the reacting substance.

According to this feature, a protein can be fluorescently labeled without making a protein incorporate a large substance other than the protein or chemically modifying the protein, a reaction experiment can be performed while the original function of a protein is maintained, and the size, brightness or count of a substance having a fluorescent label can be obtained by mixing of each solution and utilizing fluorescence analysis. Therefore, the detection result such as the presence or absence of a reaction, change in the size, brightness, or number of a protein, and promotion or inhibition of, or absence of influence on, a reaction by a substance reacting with the protein can be obtained simply, in a short time and at a higher precision, without a troublesome procedure such as utilization of a radioisotope, electrophoresis, work of immobilizing a molecule on a solid substrate, and washing work.

According to the present invention, a reaction experiment can be performed while the original function of a protein is remain unchanged, and the detection result such as the presence or absence of a reaction, change in the size, brightness, or number of a protein can be obtained simply, in a short time and at a higher precision, without a troublesome procedure such as utilization of a radioisotope, electrophoresis, work of immobilizing a molecule on a solid substrate, and washing work.

In addition, change in the size, brightness, and number of a reaction product in a floating system can be detected at an excellent sensitivity of a nM order by mixing of each solution, and utilizing fluorescence correlation spectroscopy (FCS), fluorescence cross-correlation spectroscopy (FCCS), fluorescence intensity distribution analysis (FIDA), fluorescence intensity multiple distribution analysis (FIMDA) or FIDA-polarization.

### Brief Description of Drawings

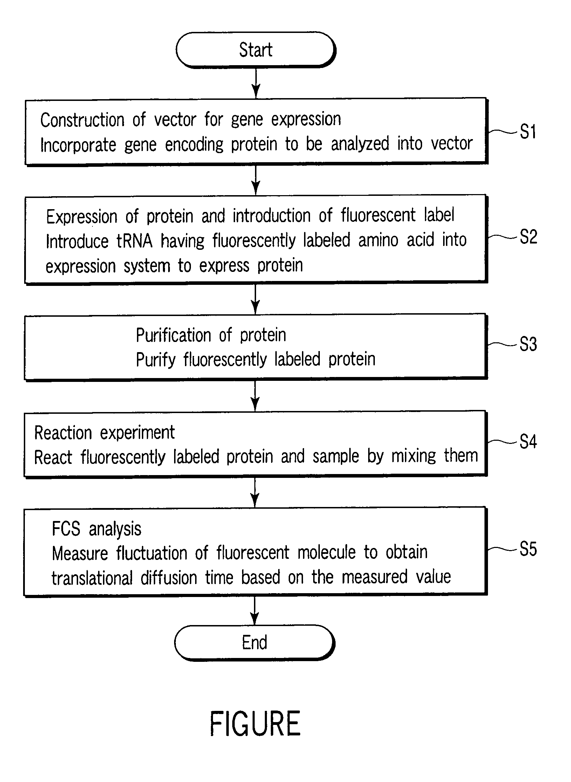

FIG. 1 is a view showing an example of a procedure of analyzing a protein in the present embodiment.

### Best Mode for Carrying Out the Invention

In the present embodiment, a protein is analyzed by the following procedure. FIG. 1 shows an example of the procedure.

### (1) Construction of vector for gene expression (S1)

A gene encoding a protein to be analyzed is incorporated into a vector having a promoter region and a terminal region.

### (2) Expression of protein and introduction of fluorescent label (S2)

By using an expression system such as an extracellular gene expression system and an extracellular transcription or translation system, a reaction of expressing a protein from the constructed vector is performed. At this time, a fluorescently labeled amino acid is introduced into the expression system, for example, in the form of a tRNA having a fluorescently labeled amino acid. With expression of the protein, the fluorescently labeled amino acid is incorporated into the protein, and a fluorescently labeled protein (hereinafter, referred to as fluorescently labeled protein) is produced.

### (3) Purification of protein (S3)

The fluorescently labeled protein is purified. Depending on an analyzing method, the protein may be used as it is without purification.

### (4) Reaction experiment (S4)

The fluorescently labeled protein and a sample S are mixed to prepare a mixture A, and then reacted under the temperature condition of a living body, for example, at 37°C. Alternatively, a substance P which is known to react with the fluorescently labeled protein may be added to the mixture A to prepare a mixture B.

### (5) FCS analysis (S5)

The reacted mixture is transferred to a glass-bottomed plate for measuring FCS, for example, a microplate, and FCS measurement is performed. Based on a measured value in the FCS measurement, the presence or absence of a reaction is detected.

In the FCS measurement, fluctuation of a fluorescent molecule in a micro region is measured, and a translational diffusion time is obtained based on the value measured. Since a magnitude of the translational diffusion time indicates a magnitude of a molecular weight, increase or decrease in the molecular weight can be obtained by comparing the translational diffusion time before and after the reaction. Increase in the molecular weight indicates a binding reaction between biological molecules, decrease in the molecular weight indicates a degradation reaction of a biological molecule, and constant molecular weight indicates the absence of both of binding and cleaving of a biological molecule. Therefore, by detecting increase in the translational diffusion time of a fluorescently labeled substance before and after a reaction between a fluorescently labeled protein and a sample, a binding reaction between the fluorescently labeled protein and the sample can be detected.

In addition, when an unpurified fluorescently labeled protein is used, change in the translational diffusion time of a protein can be analyzed by performing 2-component analysis with an unreacted fluorescently labeled tRNA.

Although, in the aforementioned procedure, fluorescence correlation spectroscopy (FCS) was used in order to obtain a translational diffusion time of a reaction product in (5), fluorescence cross-correlation spectroscopy, fluorescence intensity distribution analysis, fluorescence intensity multiple distribution analysis or FIDA-polarization may be used in place of fluorescence correlation spectroscopy. By these analysis methods, data concerning the size, number or brightness of a protein binding molecule after reaction is obtained. From these data, change in the size, number, or brightness of a molecule before and after reaction can be obtained. For example, when as a result of FCS measurement, there is not a remarkable difference in the size of a protein before and after a reaction, but there is change in the brightness of fluorescence per molecule, the presence or absence of reaction can be found by performing fluorescence intensity distribution analysis (FIDA).

Then, (4) a difference in analysis result between the mixed solutions A and B to be prepared will be explained.

### (A) Mixed solution of fluorescently labeled protein and sample S

The fluorescently labeled protein obtained in (3) and a sample S are mixed, and a reaction experiment is performed. Then, fluorescence correlation spectroscopy (FCS), fluorescence cross-correlation spectroscopy (FCCS), fluorescence intensity distribution analysis (FIDA), fluorescence intensity multiple distribution analysis (FIMDA) or FIDA-polarization is used to obtain data of the size, brightness or number regarding a fluorescently labeled reaction product. Based on these data, change in the size, brightness, or number of the reaction product before and after the reaction is obtained. When there is any change, it is detected that the fluorescently labeled protein and the sample have reacted with each other. In addition, it results in that the number of the reaction product of the fluorescently labeled protein and the sample as well as the degree of the reaction could be quantified.

### (B) Mixed solution of fluorescently labeled protein, sample S, and substance P which is known to react with fluorescently labeled protein

The fluorescently labeled protein obtained in (3), a sample S, and a substance P which is known to react with the fluorescently labeled protein are mixed, and a reaction experiment is performed. Then, fluorescence correlation spectroscopy (FCS), fluorescence cross-correlation spectroscopy (FCCS), fluorescence intensity distribution analysis (FIDA), fluorescence intensity multiple distribution analysis (FIMDA) or FIDA-polarization is used to obtain data of the size, brightness or number regarding a fluorescently labeled reaction product. Based on these data, change in the size, brightness, or number of the reaction product before and after the reaction, as well as promotion, inhibition or no change of the reaction due to the substance P are obtained. By analysing the results, reaction between the fluorescently labeled protein and the sample is accomplished. In addition, it results in that the number of the reaction product or the degree of the reaction could be quantified.

Further, by comparing change in the size, brightness, or number of the reaction product obtained when the mixed solution of (A) is used, with that of the reaction product obtained when the mixed solution of (B) is used, followings are quantified at a higher precision: a reaction of a fluorescently labeled protein and a sample, and the number of the reaction product of the fluorescently labeled protein and the sample, and the degree of the reaction.

According to the present Example, a protein can be fluorescently labeled without making the protein to be incorporated a large substance other than the protein or chemically modifying the protein, and a reaction experiment can be performed while the original function of a protein is maintained, therefore the presence or absence of a reaction, and change in the size of a protein can be detected at a higher precision.

In addition, by mixing of a fluorescently labeled protein and a sample, or other mixture and utilizing fluorescence correlation spectroscopy (FCS), fluorescence cross-correlation spectroscopy (FCCS), fluorescence intensity distribution analysis (FIDA), fluorescence intensity multiple distribution analysis (FIMDA) or FIDA-polarization, change in the size, brightness, or number of a reaction product in a floating system can be detected at an excellent sensitivity such as nM order.

In addition, the detection result can be obtained simply and in a short time without a troublesome procedure such as utilization of a radioisotope, electrophoresis, work of immobilizing a molecule on a solid substrate, and washing work. Particularly, a reaction experiment can be performed in a homogenous system in which a protein and a reactive substance are reacted while being mixed in a solution, therefore transfer of reagent can be automated, and reactors having a variety of shapes can be used. Further, a large amount of a sample can be handled on microplate at once, therefore analysis can be performed countably or continuously, simply, in a short time and at a higher precision. In addition, a solid support is not used, therefore even a protein which is difficult to be solid-phased can be reacted and analyzed.

In addition, when an automatic pipettor and a plate stacker are used, the present invention can also be applied to screening work such as an large-scale analysis conducted on large number of samples in respect to reactability (i.e. whether or not a fluorescently labeled protein is reacted with a large number of samples.

## Claims

1. A method for detecting a reaction of a protein and a sample, **characterized by** comprising: a step of synthesizing a fluorescently labeled protein using an expression system which extracellularly expresses a protein, a vector having a gene encoding the protein incorporated therein, and a fluorescently labeled amino acid; a step of mixing a solution containing the fluorescently labeled protein with a sample; and a step of obtaining a size, brightness or count of a substance having a fluorescent label in the mixed solution by a fluorescence analysis method.

2. The method for detecting a reaction of a protein and a sample according to claim 1, **characterized in that** the fluorescence analysis method is fluorescence correlation spectroscopy (FCS), fluorescence cross-correlation spectroscopy (FCCS), fluorescence intensity distribution analysis (FIDA), fluorescence intensity multiple distribution analysis (FIMDA) or FIDA-polarization.

3. A method for detecting a reaction of a protein and a sample, **characterized by** comprising: a step of synthesizing a fluorescently labeled protein using an expression system which extracellularly expresses a protein, a vector having a gene encoding the protein incorporated therein, and a fluorescently labeled amino acid; a step of mixing a solution containing the fluorescently labeled protein, a sample and a substance which reacts with the protein; and a step of obtaining a size, brightness or count of a substance having a fluorescent label in the mixed solution by a fluorescence analysis method.

4. A method for detecting a reaction of a protein and a sample, **characterized by** comprising: a step of synthesizing a fluorescently labeled protein using an expression system which extracellularly expresses a protein, a vector having a gene encoding the protein incorporated therein, and a fluorescently labeled amino acid; a step of mixing a solution containing the fluorescently labeled protein with a sample to prepare a first mixed solution; a first analysis step of obtaining a size, brightness or count of a substance having a fluorescent label in the first mixed solution by a fluorescence analysis method; a step of mixing the solution containing the fluorescently labeled protein, the sample and a substance which reacts with the protein to prepare a second mixed solution; a second analysis step of obtaining a size, brightness or count of a substance having a fluorescent label in the second mixed solution by a fluorescence analysis method; and a step of detecting promotion or inhibition of, or absence of influence on, a reaction by the reacting substance based on a size, brightness or number of the substance having a fluorescent label obtained in the first analysis step, and a size, brightness or number of the substance having a fluorescent label obtained in the second analysis step.
